# EUROPEAN PATENT APPLICATION

(11) **EP 1 527 768 A1**
(43) Date of publication of application: **04.05.2005**
(21) Application number: 03256901.4
(22) Date of filing: 31.10.2003
(51) Int. Cl.: A61K 7/06

(54) **Hair conditioning compositions**

(71) Applicant: UNILEVER PLC, London EC4P 4BQ (GB)
(72) Inventor: Dawson, Joanna Susan Unilever R&D Sunlight, Wirral Merseyside, CH63 3JW (GB); Giles, Colin Christopher David Unilever Thai Trad., Road, Ladyao, Jatujak Bangkok 10900 (TH)
(74) Representative: Tansley, Sally Elizabeth

(57) **Abstract**

Rinse-off hair conditioning compositions and methods of manufacture and use are disclosed. The compositions comprise water, cationic conditioning surfactant and a non-volatile cationic polysiloxane polymer with volatile oil. The compositions provide conditioning without loss of hair volume.

## Description

### Field of the invention

The present invention relates to rinse-off hair conditioning compositions, particularly compositions that are typically applied to the hair after cleansing with a shampoo or shower gel in order to improve the condition of the hair, particularly combability and manageability.

### Background to the Invention

Hair care compositions which provide conditioning to the hair are well know in the art. Such compositions comprise one or more conditioning agents. The purpose of the conditioning agent is to make the hair easier to comb when wet and more manageable when dry, e.g. less static and fly-away. They also make the hair feel softer. Typically, these conditioning agents are either water-insoluble oily materials which act by spreading on the hair in the form of a film, or cationic surfactant materials or polymers, which adsorb onto the hair surface.

These conditioning materials can be employed in a variety of product forms including cleansing shampoos, rinse-off conditioners (usually applied to the hair after shampooing) and leave-on products such as hair oils or serums, mousses and styling products.

Typically, water-insoluble oily conditioning materials are dispersed in aqueous products in the form of small droplets or particles in order to facilitate the stability of the dispersion to phase separation and to enhance the deposition of the oily material onto the hair.

A preferred type of water-insoluble oily conditioning material is based on silicone polymers. The use of silicones as conditioning agents in hair conditioning compositions is well known, and widely documented in the patent literature. Generally, dispersed droplets of silicone are suspended in the composition which, when applied to the hair, deposits the silicone material on the hair shaft resulting in the formation of a film. Whilst their use can give gives good conditioning of wet hair, for example wet comb properties, there is a need to further improve the conditioning behaviour on dry hair, where the use of silicones can lead to a heavy, greasy feel for some consumers. Obtaining a light, fluffy feel from the hair after treatment with a rinse-off conditioner containing cationic surfactant and silicone oils is thus highly desirable.

WO99/44565 and WO99/44567 (Unilever) disclose shampoo compositions containing a combination of an amino-functionalised silicone and an insoluble non-amino functionalised silicone.

WO97/12594 (L'Oreal) describes hair compositions containing at least one silicone-grafted polymer with a polysiloxane backbone grafted by non-silicone organic monomers and at least one silicone selected from silicones containing a quaternary amine function, silicone resins and silicone gums.

US 6,028,041 (L'Oreal) and EP 0 811 371 (L'Oreal) disclose the use of a mixture of at least one aminated silicone and at least one insoluble silicone of viscosity less than or equal to 100 Pa.s at 25°C (100,000 cSt = 100,000 mm² sec⁻¹) in conditioning, hair-washing compositions.

WO98/43599 (Unilever) discloses a hair treatment composition, such as a shampoo or conditioner, comprising a silicone component comprising (i) 0.01 to 50% by weight of a silicone gum having a viscosity greater than 1 million cSt (mm²sec⁻¹), (ii) 30 to 95% by weight of a silicone fluid having a viscosity of less than 100,000 cSt (mm²sec⁻¹), and (iii) 0.1 to 10% by weight of an amino functionalised silicone.

EP 0 811 371 discloses a detergent and hair conditioning composition comprising a washing base, at least one cationic polymer, at least one aminated silicone and at least one insoluble silicone of viscosity less than or equal to 100 Pa.s at 25°C.

Copending International application PCT/EP03/03236, discloses hair conditioning compositions which contain both an amino-functionalised silicone and a silicone with a viscosity of less than 100,000 mm²sec⁻¹.

It has now been found that by combining a silicone polymer functionalised with a quaternary Nitrogen substituent and a volatile oil in a hair conditioning base, it is surprisingly possible to achieve a rinse-off hair conditioning composition which delivers friction reduction for wet hair, giving improved wet combability, in combination with hair that feels light and fluffy when dry. In other words, conditioning obtained from a cationic surfactant-based conditioning composition can be improved, without the loss in volume of dried usually found for silicone polymers in cationic-based conditioners. Hence the invention means that there is no need for a hot-air drying step, using a hair-dryer, to obtain hair volume, and yet the hair is smooth and easily untangled when wet, prior to drying.

### Summary of the Invention

In a first aspect, the invention provides a rinse-off hair-conditioning composition comprising;
a) 40% or more by weight of water,
b) from 0.01 to 10% by weight of a cationic conditioning surfactant,
c) from 0.01 to 5% of a non-volatile cationic polysiloxane polymer with a saturated vapour pressure less than 10 Pa at 25 °C, in which at least one silicon atom of the polymer is linked to a quaternary ammonium group, and
d) from 0.01 to 10% of a volatile oil with a saturated vapour pressure of 10 Pa or more at 25 °C, selected from hydrocarbon oils, silicone oils and mixtures thereof.

A second aspect of the invention is concerned with a method for preparing a composition as described above, where the cationic polysiloxane polymer and the volatile oil are preblended into a homogeneous mixture prior to their incorporation into the composition.

A further aspect of the invention is the use of a mixture of cationic polysiloxane polymer and volatile oil to provide improved volume to dried hair from a rinse-off hair conditioner composition.

### Detailed Description

By "water-insoluble", it is meant that a component is less than 0.1% soluble by weight in water at 25 °C.

Unless otherwise specified, all percentages by weight are based upon the total weight of the composition.

The term "volatile" applied to a material here, means that the saturated vapour pressure of the material, measured at 25°C, is 10 Pa or more.

By "rinse-off hair conditioning composition" is meant a composition which is suitable for application to the hair following shampooing and rinsing while the hair is still wet. The composition is massaged into the hair followed by further rinsing prior to drying the hair.

All viscosities are measured at 25°C by using a kinematic viscosity measurement with a calibrated glass capillary following ASTM D-445 as detailed in Dow Corning Test Method CTM 0004.

### Cationic polysiloxane polymer

The cationic polysiloxane polymer has at least one silicon atom of the polymer linked by an organic linking group to a quaternary ammonium group. The cationic polysiloxane polymer is non-volatile, having a saturated vapour pressure less than 10 Pa at 25 °C. It is preferred if the cationic polysiloxane polymer is water-insoluble, meaning that its solubility in distilled water is 0.1% by weight or less.

The cationic polysiloxane polymer is present at a level of 0.05% or more, preferably 0.1% or more, more preferably 0.2% or more by weight of the composition. The cationic polysiloxane polymer is present at a level of 5% or less, preferably 4% or less, more preferably 2% or less by weight of the composition.

Suitably, the cationic polysiloxane polymer has a number average molecular weight of 1500 unified mass units or more, preferably 3000 or more, more preferably 6000 or more. The polymer is preferably linear.

Preferred cationic polysiloxane polymers are substituted polydimethylsiloxanes. It is more preferred if the cationic polysiloxane polymer is a linear polydimethysiloxane which is end functionalised, in other words a quaternary nitrogen group is linked to each of the two terminal silicon atoms of the polydimethylsiloxane chain.

The quaternary nitrogen group comprises a nitrogen atom covalently bonded to four carbon atoms. The quaternary nitrogen group may have the nitrogen as part of a ring system linked to the polydimethylsiloxane chain. The quaternary nitrogen is linked to a silicon atom of the polydimethylsiloxane chain by an organic linking group.

A preferred cationic polysiloxane polymer for use in the invention is described by the following formula: 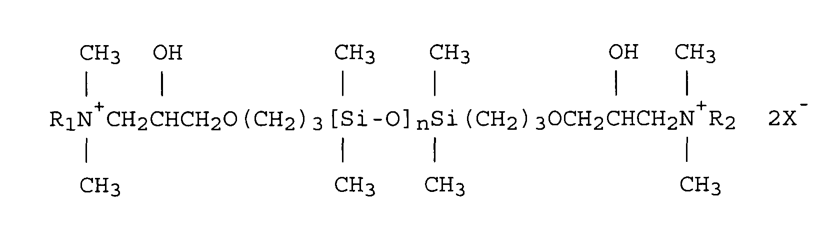 wherein R1 and R2 are independently selected from C6 to C26 saturated or unsaturated organic chains, optionally including nitrogen, oxygen, sulphur, carbon or phosphorus. Preferably R1 and R2 are alkyl amidopropyl groups. More preferably, R1 and R2 are hydrocarbon chains derived from coconut oil, preferably coco-amidopropyl groups.

In the formula, n is preferably 60 or more, more preferably 70 or more. Preferably n is 200 or less, more preferably 100 or less.

The counterion X for the quaternary nitrogen group is suitably halide, alkylsulphate such as methosulphate or preferably acetate.

A preferred quaternary silicone polymer has the CTFA (INCI) designation Quaternium-80, with R1 and R2 as coco-amidopropyl groups and with X as acetate. Such a material with n of about 80 is Abilquat 3474. It is commercially available from Degussa Care Specialities.

### Volatile Oil

Volatile oils suitable for use in compositions according to the invention have a saturated vapour pressure measured at 25°C of 10 Pa or more, preferably 100 Pa or more, more preferably 1000 Pa or more.

Suitable volatile oils include branched or straight chain, saturated or unsaturated hydrocarbons, and volatile linear or cyclic polysiloxanes. A suitable hydrocarbon oil is sold under the trade name Isopar-L.

Preferred volatile oils are linear and cyclic polysiloxanes, particularly polydimethylsiloxanes (CTFA designations dimethicone and cyclomethicone).

Examples of preferred cyclomethicones are the tetramer, pentamer and hexamer forms of cyclomethicone, and mixtures thereof. An example of a preferred linear volatile dimethicone is Dow Corning 200 fluid, 0.65 cs, which has a saturated vapour pressure of about 1300Pa at 25°C.

### Other Silicone Polymers

Compositions according to the invention may further comprise other silicone polymers such as amodimethicones, polydimethylsiloxanes or mixtures thereof. The invention in this aspect can provide additional wet conditioning without any additional reduction in hair volume.

Alternatively, the compositions according to the invention may comprise no silicone oils other than the cationic polysiloxane polymer and any silicone which is part of the volatile oil of the composition.

In this latter case other silicones are present at levels less than 0.05% by weight of the composition, preferably less than 0.01%.

### Process

One method for preparing compositions according to the invention is to first prepare a conditioning oil blend comprising the non-volatile cationic polysiloxane polymer and the volatile oil. It is preferred if the volatile oil and the cationic polysiloxane polymer are present in the composition as droplets of a homogeneous blend, the blend comprising both the non-volatile cationic polysiloxane polymer and the volatile oil. By homogeneous blend it is meant that the volatile oil and the cationic polysiloxane polymer are mixed together on a scale such that each droplet of the blend in the composition comprises both the volatile oil and the cationic polysiloxane polymer in substantially the same proportion as in the composition as a whole.

This conditioning oil blend can then be added along with the other components comprising the hair conditioning composition, followed by suitable mixing of the composition in order to ensure that the conditioning oil blend is dispersed as droplets of a suitable size.

However it is preferred if the conditioning oil blend is first formed into an aqueous emulsion prior to incorporation into the hair conditioning composition. Thus another aspect of the invention is a method for incorporating droplets of conditioning oil blend, the conditioning oil blend comprising a non-volatile cationic polysiloxane polymer and a volatile oil, into a hair conditioning composition, comprising the steps of;
i) forming a homogeneous blend comprising both the non-volatile cationic polysiloxane polymer and the volatile oil,
ii) preparing an aqueous emulsion comprising droplets of the homogeneous blend and
iii) mixing the aqueous emulsion with the hair conditioning composition;

Suitable emulsifiers for use in the preparation of the aqueous emulsion are well known in the art and include anionic, cationic, zwitterionic, amphoteric and nonionic surfactants, and mixtures thereof. It is preferred if the emulsifier is selected from cationic surfactants, nonionic surfactants and mixtures thereof, to ensure compatibility with the cationic polysiloxane polymer.

Examples of nonionic surfactants suitable for use as emulsifiers for the conditioning oil blend are alkylphenol ethoxylates, e.g., nonylphenol ethoxylate nEO, where n is from 1 to 50 and alcohol ethoxylates, e.g., lauryl alcohol nEO, where n is from 1 to 50, ester ethoxylates, e.g., polyoxyethylene monostearate where the number of oxyethylene units is from 1 to 30.

It is preferred if the emulsifier is blended into the conditioning oil blend prior to the formation of the aqueous emulsion of the mixture droplets.

A preferred process for preparing oil-in-water emulsions of the conditioning oil blend droplets which can then be incorporated into the hair treatment compositions involves use of a mixer. Depending upon the viscosities of components of the conditioning oil blend, a suitable mixer should be chosen so as to provide sufficient shear to give the required final particle size of the emulsion. Examples of suitable benchtop mixers spanning the range of necessary shear are Heidolph RZR2100, Silverson L4R, Ystral X10/20-750 and Rannie Mini-Lab 7.30VH high pressure homogeniser. Other mixers of similar specification are well known to those skilled in the art and can also be used in this application. Equally it is possible to manufacture oil-in-water emulsions of this description on larger scale mixers which offer similar shear regimes to those described above.

The required amounts of the cationic polysiloxane polymer and the volatile oil are combined under shear to produce a uniform mixture: the conditioning oil blend. To this blend, a suitable emulsifier system is added slowly with further shear. Examples of suitable emulsifier systems for this application are given above. As is well known to those skilled in the art, the emulsifier system can be used to help to control the final particle size of the emulsion.

When the addition of the emulsifier is complete the aqueous portion of the emulsion is added slowly with the required level of shear so as to produce an emulsion with the desired particle size.

It is preferred if the aqueous phase of the emulsion contains a polymeric thickening agent to prevent phase separation of the emulsion after preparation. Preferred thickening agents are cross-linked polyacrylates, cellulosic polymers or derivatives of cellulosic polymers.

Alternatively the order of addition of these materials may sometimes be varied in order to achieve the same outcome.

Preferably, the mixer is also capable of having the temperature of mixing controlled, e.g. it comprises a jacket through which a heat transfer fluid can be circulated.

Preferably, the D_{3,2} average particle size of the conditioning oil blend droplets in the emulsion and also in the final composition is from 0.01 to 20 micrometres, more preferably from 0.1 to 10 micrometres even more preferably from 1 to 5 micrometres.

Silicone particle size may be measured by means of a laser light scattering technique, for example using a 2600D Particle Sizer from Malvern Instruments.

### Cationic Conditioning Surfactant

Compositions according to the invention comprise one or more cationic conditioning surfactants which are cosmetically acceptable and suitable for topical application to the hair.

Cationic conditioning surfactants useful in compositions of the invention contain amino or quaternary ammonium hydrophilic moieties which are positively charged when dissolved in the aqueous composition of the present invention.

Examples of suitable cationic surfactants are those corresponding to formula 1:

1 [N(R₁) (R₂) (R₃) (R₄)]⁺ (X)⁻

in which R₁, R₂, R₃, and R₄ are independently selected from (a) an aliphatic group of from 1 to 22 carbon atoms, or (b) an aromatic, alkoxy, polyoxyalkylene, alkylamido, hydroxyalkyl, aryl or alkylaryl group having up to 22 carbon atoms; and X is a salt-forming anion such as those selected from halogen, (e.g. chloride, bromide), acetate, citrate, lactate, glycolate, phosphate nitrate, sulphate, and alkylsulphate radicals.

The aliphatic groups can contain, in addition to carbon and hydrogen atoms, ether linkages, and other groups such as amino groups. The longer chain aliphatic groups, e.g., those of about 12 carbons, or higher, can be saturated or unsaturated.

Preferred cationic surfactants for conditioner compositions of the present invention are those according to formula 1 in which R₁ is a C₁₆ to C₂₂ alkyl chain and R₂, R₃ and R₄ are independently selected from the group consisting of CH₃ and CH₂CH₂OH. Most preferred are surfactants with R₂, R₃ and R₄ all as methyl groups.

Another preferred class of cationic conditioning surfactants has R₁ and R₂ independently selected from C₁₆ to C₂₂ hydrocarbyl chains comprising at least one ester linkage in both R₁ and R₂, and with R₃ and R₄ independently selected from the group consisting of CH₃ and CH₂CH₂OH.

Another preferred class of cationic conditioning surfactants has R₁ and R₂ independently selected from C₁₆ to C₂₂ saturated or unsaturated, preferably saturated, chains and with R₃ and R₄ independently selected from the group consisting of CH₃ and CH₂CH₂OH, preferably CH₃.

Examples of preferred cationic surfactants used alone or in admixture include:
cetyltrimethylammonium chloride and behenyltrimethylammonium chloride and dihydrogenated tallow dimethylammonium chloride.

Salts of primary, secondary, and tertiary fatty amines are also suitable cationic surfactants. The alkyl groups of such amines preferably have from about 12 to about 22 carbon atoms, and can be substituted or unsubstituted.

Particularly useful are amido substituted tertiary fatty amines. An example of such an amine is stearamidopropyIdimethylamine. These amines are typically used in combination with an acid to provide the cationic species. The preferred acid useful herein includes L-glutamic acid, lactic acid, hydrochloric acid, malic acid, succinic acid, acetic acid, fumaric acid, tartaric acid, citric acid, L-glutamic hydrochloride, and mixtures thereof; more preferably L-glutamic acid, lactic acid, citric acid. Cationic amine surfactants included among those useful in the present invention are disclosed in U.S. Patent 4,275,055 to Nachtigal.

The molar ratio of protonatable amines to H⁺ from the acid is preferably from about 1:0.3 to 1:1.2, and more preferably from about 1:0.5 to about 1:1.1.

In the conditioners of the invention, the level of cationic surfactant is preferably from 0.01 to 10, more preferably 0.05 to 5, most preferably 0.1 to 2 % by weight of the total composition.

### Fatty Materials

Conditioner compositions of the invention preferably additionally comprise fatty materials. The combined use of fatty materials and cationic surfactants in conditioning compositions is believed to be especially advantageous, because this leads to the formation of a lamellar phase, in which the cationic surfactant is dispersed.

By "fatty material" is meant a fatty alcohol, an alkoxylated fatty alcohol, a fatty acid or a mixture thereof.

Preferably, the alkyl chain of the fatty material is fully saturated.

Representative fatty materials comprise from 8 to 22 carbon atoms, more preferably 16 to 22. Examples of preferred fatty alcohols include cetyl alcohol, stearyl alcohol, behenyl alcohol and mixtures thereof. The use of these materials is also advantageous in that they contribute to the overall conditioning properties of compositions of the invention.

Alkoxylated, (e.g. ethoxylated or propoxylated) fatty alcohols having from about 12 to about 18 carbon atoms in the alkyl chain can be used in place of, or in addition to, the fatty alcohols themselves. Preferred examples include ethylene glycol cetyl ether, polyoxyethylene (2) stearyl ether, polyoxyethylene (4) cetyl ether, and mixtures thereof.

The level of fatty alcohol material in conditioners of the invention is suitably from 0.01 to 15, preferably from 0.1 to 10, and more preferably from 0.1 to 5% by weight of the composition. The weight ratio of cationic surfactant to fatty alcohol is suitably from 10:1 to 1:10, preferably from 4:1 to 1:8, more preferably from 1:1 to 1:7.

### Mousses

Hair conditioning compositions in accordance with the invention may also be used as the aqueous part of a mousse-forming composition, where the mousse-forming composition consists of a hair conditioning composition according to the invention combined in an aerosol container with a propellant. The propellant is responsible for expelling the hair conditioning composition from the container and forming the hair mousse character.

The propellant gas can be any liquefiable gas conventionally used for aerosol containers. Examples of suitable propellants include dimethyl ether, propane, n-butane and isobutane, used singly or in admixture.

The amount of the propellant gases is governed by normal factors well known in the aerosol art. For hair mousses, the level of propellant is generally from 3 to 30, preferably from 5 to 15 percent by weight of the total mousse composition comprising propellant and hair conditioning composition.

### Use

The composition of the invention are used by applying them to wet hair, typically hair which has been washed and then rinsed with water. The compositions are massaged into the hair followed by further rinsing with water, hence the name "rinse-off" hair conditioning composition.

Another aspect of the invention is the use of compositions as described above to deliver friction reduction for wet hair, giving improved wet combability, in combination with hair that feels light and fluffy when dry. Another use of the invention is to increase the volume of dry hair, without the need for a hot-air drying step using a hair-dryer, and yet the hair is smooth and easily untangled when wet, prior to drying.

The invention will now be further described by reference to the following examples.

### Examples

**Table 1**

| **Active** | **Tradename** | **Weight Percent of active material** |
|---|---|---|
| dihydrogenated tallow dimethyl ammonium chloride | Arquad 2HT75 | 1.6 |
| Cetearyl trimonium chloride | CTAC 16/50 | 0.8 |
| Cetostearyl alcohol | Laurex CS | 5 |
| Methyl Paraben | Nipagen M | 0.2 |
| Conditioning blend | - | - |
| Water | | to 100 |

Various examples were prepared using as a base formulation the formula detailed in table 1. Compositions were prepared by heating water and CTAC to 80°C into a clear solution while separately melting together the Laurex and the Arquad. The melt was then stirred into the solution and the resulting mix cooled to 25°C before adding the remaining ingredients, including the conditioning blend.

In the Examples, DC245 is a volatile cyclomethicone with a vapour pressure of about100 Pa at 25°C. DC8830 is an aminosilicone (CTFA designation amodimethicone) and DC200 - 300,000 cSt is a polydimethylsiloxane of kinematic viscosity 300,000 mm²sec⁻¹ at 25 °C.

Three examples were prepared as follows:
A Base as in table 1 without conditioning blend
   1 Base with, as conditioning blend, 1% Abilquat 3474 + 4% Silicone DC245 added as separate ingredients.
   2 Base with, as conditioning blend, 1% Abilquat 3474 + 4% Silicone DC245 added as a pre-formed blend.

Hair Volume was measured as follows: 2g, 25 cm long Asian hair switches were washed using a dilute aqueous solution of 16:2 by weight sodium lauryl ether sulphate:coco amidopropyl betaine solution then rinsed in water at 35-40 °C. Excess water was removed by running thumb and forefinger along he switches. Using 5 switches per treatment, 2 ml of a conditioner composition was spread along the length of the switches and agitated for 1 minute, followed by a rinse in running 35-40°C water for 1 minute. The switches were combed through whilst suspended vertically from a clamp stand, then smoothed with thumb and forefinger. The switches were then allowed to dry naturally overnight.

After drying, each switch was suspended vertically from a clamp stand and a 2 mW, 632.8 nanometre wavelength Helium-Neon laser shone perpendicular to the untouched switch, 5 cm from the bottom of the switch, and the illuminated image recorded onto an optical disc using a 35mm camera.

Image analysis was carried out on the resulting image to estimate the spread of each hair switch 5 cm from the bottom of each switch (expressed as mean radial distribution in mm). The smaller the value for the spread, the lower the apparent volume of the switch and the greater the straightness of the fibres.

The friction for wet or dry hair was measured using a laboratory technique with a commercially available texture analyser TA XT2i ex Stable Microsystems.

Table 2 shows the results for examples A (comparative) and 1 and 2 (according to the invention).

**Table 2**

| Example | Wet Friction | Volume |
|---|---|---|
| A | 23.9 | 17.5 |
| 1 | 19.8 | 18.3 |
| 2 | 19.9 | 16.1 |

From the results it can be seen that examples 1 and 2 give a good reduction in wet friction while having a relatively small effect on the hair volume. This can be compared with the volume effects arising from the following examples which gave good reduction in wet friction:
B: 1% DC 8830 (amodimethicone) premixed with DC245 (1:4 ratio). Volume 14.0
C: 1% Abilquat 3474 premixed with DC200 silicone (300,000 mm² sec⁻¹ at 25°C). Volume 12.0

The results for B and C show that the combination of both quaternary silicone and volatile oil is needed to avoid loss of volume; either on its own is not adequate.

## Claims

1. A rinse-off hair-conditioning composition comprising;
a) 40% or more by weight of water,
b) from 0.01 to 10% by weight of a cationic conditioning surfactant,
c) from 0.01 to 5% of a non-volatile cationic polysiloxane polymer with a saturated vapour pressure less than 10 Pa at 25 °C, in which at least one silicon atom of the polymer is linked by an organic linking group to a quaternary ammonium group, and
d) from 0.01 to 10% of a volatile oil with a saturated vapour pressure of 10 Pa or more at 25 °C, selected from hydrocarbon oils, silicone oils and mixtures thereof.

2. A composition according to claim 1 in which a quaternary nitrogen group is linked by an organic linking group to each of the two terminal silicon atoms of the polysiloxane chain

3. A composition according to any preceding claim in which the cationic polysiloxane polymer is a cationic polydimethylsiloxane polymer.

4. A composition according to any preceding claim in which the volatile oil is a linear polydimethylsiloxane.

5. A composition according to any preceding claim in which the volatile oil is a cyclic polydimethylsiloxane.

6. A composition according to any preceding claim wherein the volatile oil and the cationic polysiloxane polymer are present in the composition as droplets of a homogeneous blend, the blend comprising both the non-volatile cationic polysiloxane polymer and the volatile oil.

7. A composition according to any preceding claim in which the cationic conditioning surfactant is selected from the group consisting of cetyl trimethylammonium chloride, stearyltrimethylammonium chloride, behentrimonium chloride and mixtures thereof.

8. A composition according to any preceding claim in which the cationic polysiloxane polymer is Quaternium-80.

9. A method for incorporating droplets of a conditioning oil blend, the conditioning oil blend comprising a non-volatile cationic polysiloxane polymer and a volatile oil, into a rinse-off hair conditioning composition according to claim 1, comprising the steps of;
i) forming a homogeneous conditioning oil blend comprising both the non-volatile cationic polysiloxane polymer and the volatile oil,
ii) preparing an aqueous emulsion comprising droplets of the homogeneous conditioning oil blend wherein each droplet comprises both the non-volatile cationic polysiloxane polymer and the volatile oil and
iii) mixing the aqueous emulsion with any other ingredients of the hair conditioning composition;
